# FASCICULE DE BREVET EUROPEEN

(11) **EP 2 349 272 B1**
(45) Date de publication et mention de la délivrance du brevet: **16.01.2019**
(21) Numéro de dépôt: 09783801.5
(22) Date de dépôt: 07.10.2009
(51) Int. Cl.: A61K 31/48, A61P 5/08

(54) **COMPOSITION VETERINAIRE ANTIPROLACTINIQUE DESTINEE AUX RUMINANTS**
TIERÄRZTLICHE ANTIPROLACTIN-ZUSAMMENSETZUNG FÜR WIEDERKÄUER
ANTIPROLACTINIC VETERINARY COMPOSITION FOR RUMINANTS

(30) Priorité: 07.10.2008 FR 0805544
(43) Date de publication de la demande: 03.08.2011
(73) Titulaire: CEVA Santé Animale SA, 33501 Libourne Cedex (FR)
(72) Inventeur: LAGARDE, Anouck, F-33270 Floirac (FR); FLOCH, Stephane, F-33133 Galgon (FR); BERTAIM, Thierry, F-33230 Coutras (FR)
(74) Mandataire: Becker, Philippe
(86) Numéro de dépôt international: PCT/EP2009/063004
(87) Numéro de publication internationale: WO 2010/040765

(56) Documents cités:
- EP-A- 1 952 813
- WO-A-00/54776
- WO-A-03/043653
- WO-A-2004/113378
- FORSYTH ISABEL A ET AL: "Bromocriptine treatment of periparturient goats: Long-term suppression of prolactin and lack of effect on lactation" JOURNAL OF DAIRY RESEARCH, vol. 60, no. 3, 1993, pages 307-317, XP009115443 ISSN: 0022-0299
- DATABASE BIOSIS [Online] BIOSCIENCES INFORMATION SERVICE, PHILADELPHIA, PA, US; 1985, FORSYTH I A ET AL: "HORMONE CONCENTRATIONS MAMMARY DEVELOPMENT AND MILK YIELD IN GOATS GIVEN LONG-TERM BROMOCRIPTINE TREATMENT IN PREGNANCY" XP002524367 Database accession no. PREV198579089021 & JOURNAL OF ENDOCRINOLOGY, vol. 104, no. 1, 1985, pages 77-83, ISSN: 0022-0795
- PETERSON S W ET AL: "Long-term bromocriptine treatment during late pregnancy has differential effects on milk yields of single- and twin-bearing ewes" NEW ZEALAND JOURNAL OF AGRICULTURAL RESEARCH, vol. 40, no. 2, 1997, pages 249-259, XP009115439 ISSN: 0028-8233
- DATABASE EMBASE [Online] ELSEVIER SCIENCE PUBLISHERS, AMSTERDAM, NL; 1981, AKERS R M ET AL: "Prolactin regulation of cytological differentiation of mammary epithelial cells in periparturient cows" XP002524368 Database accession no. EMB-1981202653 & ENDOCRINOLOGY 1981 US, vol. 109, no. 1, 1981, pages 31-40, ISSN: 0013-7227
- LEITNER ET AL: "Casein hydrolyzate intramammary treatment improves the comfort behavior of cows induced into dry-off" LIVESTOCK SCIENCE, ELSEVIER, AMSTERDAM, NL, vol. 110, no. 3, 1 juillet 2007 (2007-07-01), pages 292-297, XP022133134 ISSN: 1871-1413
- DATABASE BIOSIS [Online] BIOSCIENCES INFORMATION SERVICE, PHILADELPHIA, PA, US; 1995, BUYS N ET AL: "Bromocriptine is effective in reducing milk production in ewes during lactation, but has no additional effect during drying off" XP002555205 Database accession no. PREV199598275520 & BUYS N ET AL: "Bromocriptine is effective in reducing milk production in ewes during lactation, but has no additional effect during drying off" 1995, ANIMAL SCIENCE (PENCAITLAND), VOL. 60, NR. 2, PAGE(S) 203-208

## Description

La présente invention a pour objet une composition vétérinaire antiprolactine destinée à être administrée aux ruminants. Cette composition comprend de la cabergoline en tant que composé antiprolactinique agoniste des récepteurs de la dopamine, et est particulièrement utile pour favoriser l'involution mammaire, la régénération du tissu mammaire et les conditions de tarissement de la lactation. Les compositions selon la présente invention sont en outre administrées en période de gestation car elles ne présentent pas d'effet délétère ou abortif sur la gestation des ruminants.

Chez les ruminants la lactation dure de 5 à 20 mois en général, elle est de 10 mois en moyenne chez la vache laitière. Après cette période de lactation, on arrête en général brutalement la traite, et la vache est mise au repos pendant une période qui dure classiquement 60 jours (dite période sèche) jusqu'au vêlage, point de départ de la lactation suivante. Dans les élevages laitiers, il est reconnu que cette période sèche améliore sensiblement la santé et le bien être des ruminants. Cette mise au repos physiologique de la mamelle est nécessaire pour la régénération de la glande mammaire et la préparation des futures lactations. Elle limite également l'apparition de troubles de la santé à l'arrêt de la traite et autour du vêlage, tels que notamment l'apparition d'inflammations et d'infections des mamelles qui ont un impact économique important.

Des composés antiprolactiniques sont prescrits en médecine humaine en tant que traitement des hyperprolactinémies, des adénomes hypophysaires, de la maladie de Parkinson, ou bien encore en vue de provoquer un arrêt de la lactation chez certaines femmes qui ne peuvent pas allaiter pour des raisons médicales. De tels médicaments contiennent comme principes actifs des inhibiteurs de la sécrétion hypophysaire de prolactine, une hormone qui active la sécrétion lactée. Il s'agit par exemple de médicaments comme le Parlodel® (bromocriptine, Novartis), le Dostinex® ou le Cabaser® (cabergoline, Pfizer) pour lesquels il est explicitement précisé qu'ils ne peuvent en aucun cas être pris pendant la grossesse du fait de leurs effets délétères sur le foetus. Lors d'une prescription pour stopper l'allaitement, il est recommandé de les administrer immédiatement après l'accouchement afin de stopper la montée de lait dans les 24h.

En médecine vétérinaire, des compositions à base d'inhibiteur de la prolactine, telles que le Galastop® (cabergoline, Ceva Santé Animale) peuvent être en outre administrées en tant que traitements des lactations de pseudo-gestations chez les chattes ou les chiennes. Elles sont aussi prescrites suite à un sevrage précoce, à des avortements, ou lors du retrait immédiat de la portée après la parturition. Chez les animaux, comme dans le cas des patients humains, les inhibiteurs de la sécrétion de prolactine ont toujours été prescrits en dehors de la gestation ou de la grossesse du fait des effets de toxicité, des risques de malformations du foetus, ainsi que des risques d'avortements spontanés.

Chez les mammifères ruminants, il est établi que la prolactine joue un rôle important pour la maturation de la glande mammaire et le démarrage de la lactation après vêlage mais ne présente pas d'action sur la production de lait lors de la lactation établie (Karg H. & Shams D., J. Reprod Fertil., 1974 Aug ; 39(2) ;463-72 - Shams et al., Experentia, 1972 ; (28) :697-699 - Akers et al., Endocrinology, 1981 ; (109) :23-30 - Plant K. et al., Domest Anim Endocrinol, 1987 ; (4) ; 279-290 - Knight , Livestock Production Science, 2001, (70) ; 87-93 - Dalh et al, J ;Anim. Sci., 2003 ; (81), supl3 ; 11-17). G. Leitner et al., Livestock Science 110 (2007) 292-297 décrivent l'utilisation d'hydrolysats de caséine pour améliorer le bien-être des vaches laitières au moment de la cessation de la traite.

Contrairement à bon nombre de ces publications qui enseigne que la diminution de la prolactine n'a pas d'action ou une très faible action sur la production de lait lors de la lactation établie des principales espèces laitières, telles que les vaches et les chèvres, la Demanderesse a découvert de manière surprenante que l'administration unique d'une dose d'un composé antiprolactinique agoniste des récepteurs de la dopamine tel que la cabergoline, déclenchait une baisse substantielle de la lactation et permettait d'induire efficacement la phase initiale du tarissement qui consiste en l'involution mammaire.

Egalement et contre toute attente, la Demanderesse a découvert que des compositions vétérinaires comprenant des composés antiprolactiniques agonistes des récepteurs de la dopamine pouvaient être administrées aux ruminants afin de d'induire la baisse de la lactation, de favoriser l'involution mammaire et les conditions de tarissement tissu, et ceci même pendant la gestation sans toutefois entrainer d'effets délétères ou abortifs.

L'administration de ces compositions vétérinaires aux ruminants s'est en effet révélée particulièrement avantageuse puisqu'elle a permis de réduire significativement la lactation et de favoriser l'involution mammaire, et ceci même pendant la gestation, résultant ainsi en une amélioration des conditions du tarissement. Les compositions vétérinaires selon l'invention assurent en outre une amélioration notable de la santé du pis, une meilleure régénération des tissus sécréteurs endommagés dans le pis, ainsi qu'une réduction des fréquences des maladies et/ou infections de la mamelle ou des mammites des ruminants au cours des lactations suivantes.

### Résumé de l'invention

La présente invention concerne des compositions vétérinaires comprenant de la cabergoline administrées à des ruminants en gestation pour une utilisation pour induire un tarissement de la lactation et favoriser l'involution mammaire de manière à favoriser leur bien-être ou à traiter et/ou prévenir des maladies et/ou infections de leurs mamelles.

Selon l'invention, ces compositions sont administrées en quantités thérapeutiques efficaces, notamment en traitement unique, de sorte à induire la baisse de la lactation. Egalement, ces compositions peuvent même être administrées, à des ruminants gestants sans entraîner d'effet délétère ou abortif sur la gestation. Elles favorisent ainsi de manière significative le bien être des ruminants, et sont particulièrement utiles dans l'involution mammaire, le traitement et/ou la prévention des inflammations et infections des mamelles des ruminants. La présente invention a en outre pour objet des méthodes pour favoriser l''involution mammaire chez les ruminants, ainsi que des méthodes de prévention et/ou de traitement des infections des mamelles des ruminants comme par exemple les mammites. La présente invention a enfin pour objet des kits visant à favoriser l'involution mammaire, et la baisse de la production de lait des ruminants, ainsi que des kits de traitement et/ou de prévention des mammites desdits ruminants.

### Brève description des Figures

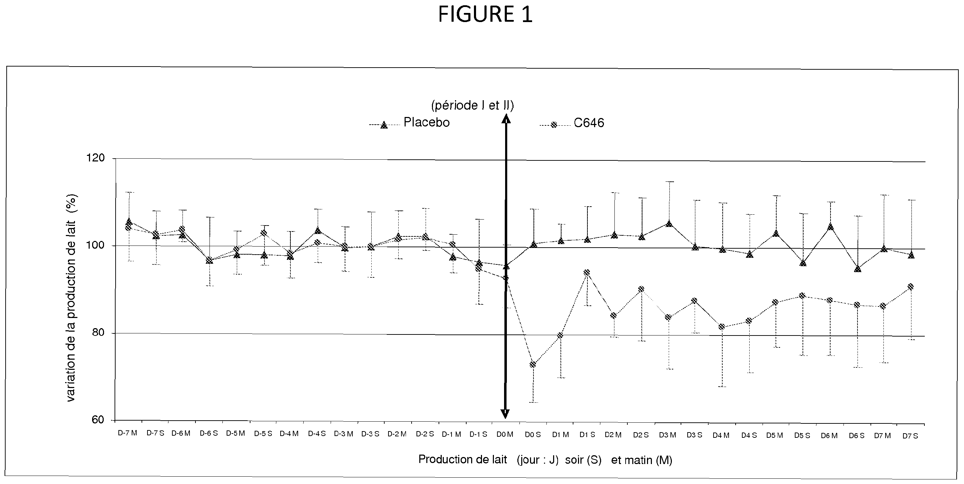

**Figure 1** **:** est un graphe montrant la variation en pourcentage de la production de lait matin et le soir entre Jour J-7 et jour J7 (par rapport à une ligne de base de 100) suite à une injection unique de 5mcg/kg de Cabergoline au jour J0, dans un groupe placebo et dans un goupe des animaux traités (C646).

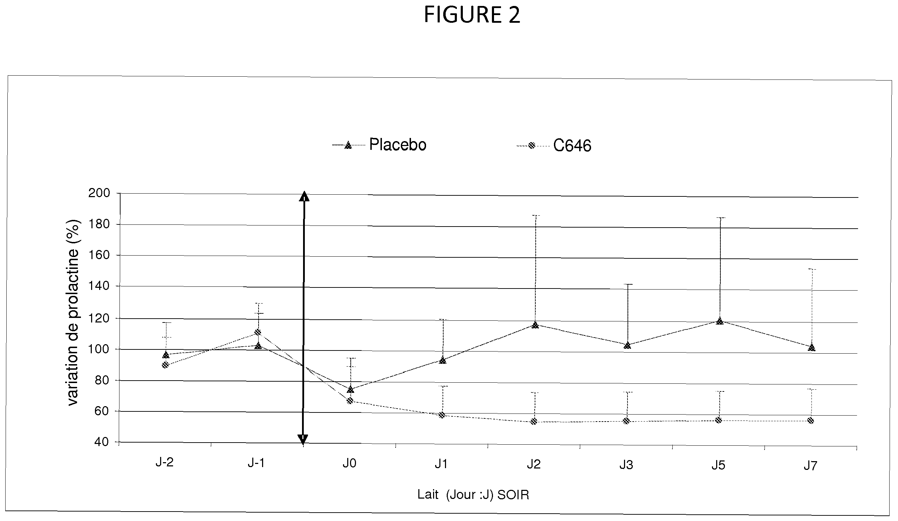

**Figure 2** **:** est un graphe qui représente la variation en pourcentage du taux de prolactine observé toutes les 24 h pendant la période I (J-2 et J-1) et la période II (entre J0 et J7), par rapport à une ligne de base de 100, suite à une injection unique de 5mcg/kg de Cabergoline au jour J0, dans un groupe placebo et dans un goupe des animaux traités (C646).

### Description détaillée de l'invention

La présente invention a pour objet une composition vétérinaire comprenant de la cabergoline administrée en une seule dose afin d'induire le tarissement et une baisse substantielle de la lactation chez l'animal traité dès la première traite ainsi que la traite suivante. La présente invention a pour objet l'utilisation de ladite composition vétérinaire administrée en quantité thérapeutique suffisante et en une seule dose, pour induire une baisse substantielle de la lactation.

La prolactine est une hormone hypophysaire qui a un effet mammotrope et lactogénique c'est-à-dire qu'elle active la croissance des glandes mammaires et la sécrétion du lait. La libération de la prolactine est sous influence stimulatrice de la prolactolibérine et inhibitrice de la dopamine. L'action inhibitrice de la dopamine au niveau de l'hypophyse est médiée par des récepteurs post-synaptiques de la dopamine, tels que notamment le récepteur D2.

Les composés sont dits antiprolactiniques lorsqu'ils inhibent la libération de la prolactine. Il peut s'agir de composés dopaminergiques ou de composés sérotoninergiques. Les composés dopaminergiques sont des agonistes des récepteurs de la dopamine, susceptibles de se lier aux récepteurs de la dopamine présents notamment au niveau des cellules sécrétrices de la prolactine de l'antéhypophyse afin d'inhiber la sécrétion de prolactine.

De préférence, ces composés antiprolactiniques dopaminergiques se lient aux récepteurs de la dopamine. De manière encore plus préférentielle, ces composés se lient de manière spécifique aux récepteurs D2 de la dopamine. Les composés antiprolactiniques antisérotoninergiques agissent en stimulant la libération de dopamine dans l'hypothalamus, aboutissant ainsi à une inhibition de la sécrétion de prolactine. Les compositions vétérinaires selon la présente invention comprennent de la cabergoline en tant que composé antiprolactinique dopaminergique.

La cabergoline dont le nom chimique est le N-[3-(Dimethylamino)propyl]-N-[(ethylamino)carbonyl]- 6-(2-propenyl)-8g-ergoline-8-carboxamide, est un composé antiprolactinique agoniste spécifique des récepteurs D2 de la dopamine. Il est notamment décrit dans le brevet US 4,526,892. Sa formule chimique développée est la suivante :

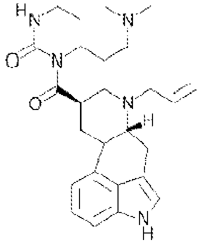

La cabergoline constitue le principe actif des médicaments humains commercialisés sous les dénominations Dostinex® et Cabaser®. Egalement, c'est le principe actif de base des compositions vétérinaires commercialisées sous la dénomination Galostop® destinées aux chattes ou aux chiennes sujettes à des lactations de pseudo-gestation. Que ce soit le Dostinex® ou le Galostop®, ces compositions à base de cabergoline ne sont jamais administrées pendant la grossesse ou la gestation.

La cabergoline a jusqu'à présent toujours été administrée en dehors de la grossesse ou de la gestation.

La Demanderesse a toutefois constaté de manière surprenante que ce composé lorsqu'il était administré à des ruminants, en administration unique et en quantité thérapeutique efficace, induisait en fait une baisse significative de la lactation, pour favoriser l'involution mammaire et améliorer le tarissement de la lactation. Contrairement à ce qui était connu jusqu'à présent, ce composé s'est avéré particulièrement bénéfique puisqu'il permettait de tarir de manière efficace la production de lait notamment pendant la gestation des ruminants sans provoquer d'effets délétères et abortifs. Enfin, le composé selon l'invention assurait une diminution significative des risques d'apparition des mammites des ruminants ainsi traités.

Comme cela est démontré dans les exemples ci-dessous, une seule dose peut être administrée aux animaux non humains afin de favoriser l'involution mammaire et les conditions du tarissement de la lactation, que ceux-ci soient en période de gestation ou après vêlage. Les quantités ou doses thérapeutiques efficaces sont susceptibles de varier en fonction des ruminants à traiter et selon le mode d'administration des compositions. Les dosages peuvent facilement être déterminés par des essais systématiques sur la base des exemples ci-dessous à la portée de l'homme du métier. A titre d'exemples, les doses thérapeutiques efficaces selon la présente invention sont comprises entre 5 et 50mcg/kg, ou entre 5 et 25 mcg/kg.

De préférence selon l'invention, les compositions sont administrées en dose unique par traitement et par animal de sorte à obtenir une baisse substantielle de la lactation comprise entre 5 et 60%, entre 20 et 50%, ou encore entre 25 et 35%, sur une durée allant de 1 jour, 2 jours, 3 jours, 4 jours, 5 jours, 6 jours et jusqu'à 7 jours après l'administration de la composition. Comme cela est démontré dans l'Exemple 2, la baisse de la lactation est significative dès la première traite, mais également lors de la traite suivante.

Il est possible d'administrer les compositions selon l'invention aux ruminants en gestation et plus précisément au cours des deux derniers tiers de la gestation, sans toutefois entrainer d'effets délétères ou abortifs. A titre d'exemple, lesdites compositions vétérinaires selon l'invention peuvent être administrées à partir du 3^{ième} mois et plus de la gestation des vaches laitières. De préférence, elles peuvent être administrées aux ruminants gestants entre le 3^{ième} mois et le 8^{ième} mois de gestation

Selon l'invention, on entend par ruminants, les mammifères herbivores tels que par exemple les bovins, les ovins, les caprins, les camélidés, ou les bovidés. Les compositions selon l'invention sont administrées aux mammifères ruminants producteurs de lait, tels que de préférence les vaches laitières et les brebis.

Selon l'invention, les compositions peuvent être administrées pendant la période de gestation ou en dehors de la gestation. Elles favorisent la baisse de la lactation et agissent sur la succession de bouleversements hormonaux, morphologiques et physiologiques qui affectent notamment la mamelle lors du tarissement, et notamment dès la phase initiale du tarissement, l'involution mammaire, au cours de laquelle le tissu sécrétoire se désorganise et s'atrophie. De manière surprenante, aucun effet délétère de toxicité, de malformations des foetus ou encore aucun effet abortif n'a été constaté lorsqu'elles sont administrées pendant la gestation des ruminants.

Les compositions vétérinaires peuvent être administrées selon toutes voies d'administration bien connues dans le domaine et adaptées au traitement de chacun des animaux. De préférence, elles sont administrées par voies cutanée, orale et parentérale. De manière encore plus préférentielle, elles sont administrées par voie parentérale, et notamment par injection intramusculaire ou sous-cutanée. Elles se présentent alors sous toutes les formes appropriées selon les modes d'administration souhaités. Elles peuvent donc être sous la forme d'une solution ou d'une suspension liquide orale ou injectable, ou sous la forme solide ou semi-solide, de poudres, de comprimés, de capsules, de granules, de dragées, de gélules, de sprays, de cachets, de pilules, de tablettes, ou de pâtes.

De manière avantageuse, la composition vétérinaire sera administrée en une seule dose injectable.

Selon les formulations des compositions et médicaments utilisées, celles-ci peuvent comprendre en outre des ingrédients conventionnellement utilisés en pharmacie pour la préparation des formulations liquides ou solides pour une administration par voie orale ou parentérale. Par ailleurs, dans le cas de formulations orales, celles-ci peuvent être administrées directement aux ruminants ou bien mélangées à la nourriture.

Egalement, les compositions selon l'invention peuvent comprendre selon le type de formulations, un solvant, un agent d'écoulement, un lubrifiant et tout excipient de masse convenable, tel que le lactose, la cellulose ou les amidons. Comme lubrifiant, on peut utiliser l'acide stéarique, le stéarate de magnésium, la L-leucine ou, par exemple, le tribéhénate de glycérol. Comme agent de désintégration, on peut utiliser le carboxyméthylamidone sodique, la carboxyméthylcellulose sodique réticulée, ou la polyvinylpyrrolidone réticulée. Comme agent d'écoulement, on peut utiliser la silice pure ou le dioxyde de silicium colloïdal. Les formes orales solides peuvent être sous formes de comprimés recouverts d'un enrobage.

Les préparations injectables sont préparées par mélange de quantités thérapeutiques efficaces d'au moins un composé antiprolactinique tel que précédemment décrit avec un solvant, un régulateur de pH, un agent tampon, un agent de mise en suspension, un agent de solubilisation, un stabilisant, un agent de tonicité et/ou un conservateur, et par transformation du mélange en une injection sous-cutanée ou intramusculaire selon un procédé classique. Comme solvant, on peut citer les solvants huileux tels que les triglycérides à chaine moyenne en C₈-C₁₀, ou un mélange d'acide caprique, d'acide caprylique et de triglycérides, tels que ceux qui sont commercialisés sous la désignation Mygliol® 812. Le cas échéant, les préparations injectables peuvent être lyophilisées selon un procédé classique. Des exemples d'agents de mise en suspension englobent la méthylcellulose, le polysorbate 80, l'hydroxyéthylcellulose, la gomme de xanthane, la carboxyméthycellulose sodique et le monolaurate de sorbitane polyéthoxylé. Des exemples d'agent de solubilisation englobent l'huile de ricin solidifiée par du polyoxyéthylène, le polysorbate 80, le nicotinamide, le monolaurate de sorbitane polyéthoxylé, le macrogol et l'ester éthylique d'acide gras d'huile de ricin. En outre, le stabilisant englobe le sulfite de sodium, le métalsulfite de sodium et l'éther, tandis que le conservateur englobe le p-hydroxybenzoate de méthyle, le p-hydroxybenzoate d'éthyle, l'acide sorbique, l'alcool benzylique, le phénol, le crésol et le chlorocrésol. Un exemple d'agent de tonicité est le mannitol. Lors de la préparation des solutions ou suspensions injectables, il est souhaitable de veiller à ce qu'elles soient isotoniques au sang.

De manière avantageuse, les compositions ou médicaments vétérinaires selon l'invention peuvent être administrés en association avec les traitements standards des mammites des ruminants. A titre d'exemples de traitements standards ou de compositions prophylactiques des mammites, on peut citer les compositions locales désinfectantes des trayons, des antibiotiques tels que les pénicillines du groupe M, les céphalosporines, la gentamycine ou la colistine, ou encore des enzymes telles que le lysozyme ou la muramidase.

La présente invention a par ailleurs pour objet l'utilisation de quantités thérapeutiques efficaces d'au moins un composé antiprolactinique agoniste des récepteurs de la dopamine tel que précédemment décrit pour la préparation de compositions ou médicaments vétérinaires destinés à être administrés aux ruminants pendant ou hors période de gestation en vue de d'améliorer leur bien être et leur santé, de favoriser le tarissement de la production de lait afin notamment, de favoriser l''involution mammaire, et de prévenir et/ou traiter les inflammations ou infections des mamelles. Lorsque les compositions ou médicaments vétérinaires sont administrés pendant la période de gestation à des doses thérapeutiques compatibles au maintien de la gestation, ceux-ci ne présentent pas d'effets délétères ou abortifs.

La présente invention concerne en outre des méthodes de tarissement de la production du lait ainsi que des méthodes de traitement et/ou de prévention des mammites comprenant l'administration par voie cutanée, orale ou parentérale à des ruminants des compositions vétérinaires précédemment décrites dans des quantités thérapeutiques efficaces. Les méthodes selon la présente invention sont particulièrement avantageuses puisqu'elles favorisent l'involution mammaire et le tarissement de la lactation des ruminants même gestants sans entrainer d'effets délétères et abortifs.

La présente invention a par ailleurs pour objet un kit à usage vétérinaire destiné à favoriser la baisse de la production de lait des ruminants, à améliorer le bien être et la santé de ces ruminants, ainsi qu'à favoriser l'involution mammaire, traiter et/ou prévenir les occurrences liées aux maladies des pis et notamment les mammites. Les kits selon la présente invention comportent au moins un compartiment pour un conditionnement éventuellement stérile comprenant une quantité thérapeutique efficace de cabergoline en tant que un composé antiprolactinique agoniste des récepteurs de la dopamine tel que précédemment décrit, pour une administration aux ruminants. Le kit comprend les moyens permettant l'administration des compositions par voie cutanée, orale ou parentérale ainsi qu'une fiche d'instructions concernant le mode d'administration des compositions ou médicaments vétérinaires selon l'invention.

### EXEMPLES

### Exemple 1 : Préparation de compositions vétérinaires à base de cabergoline

Préparation de 9 litres d'une préparation pour solution injectable contenant 500 mcg/ml de cabergoline.

### Formule :

- Principe actif : 4,53 g de cabergoline (titrant 100 %)
- Excipient : triglycérides à chaîne moyenne q. s. 9 l.

**Etape 1 :** 4,53 g de cabergoline et 1,5 kg de triglycérides à chaîne moyenne ont été mesurés dans un récipient de capacité adéquate, puis le mélange a été agité à l'aide d'un agitateur magnétique (500 tours minute) pendant au moins 60 minutes pour une dissolution complète.

**Etape 2 :** dans un récipient sec, 5 kg de triglycérides à chaîne moyenne ont été mesurés, le flux d'azote et le mélangeur ont été mis en route, puis la cabergoline en solution concentrée, obtenue à l'étape 1, a été ajoutée dans le récipient. L'agitation a été maintenue pendant 30 minutes puis le volume a été porté au volume final de 9 litres en ajoutant des triglycérides à chaîne moyenne. L'agitation a été encore maintenue 30 minutes puis la solution a été filtrée sous azote pressurisé au travers d'une cartouche calibrée à 0,45 microns. Le filtrat a été collecté dans un bidon approprié préalablement désinfecté à la vapeur et séché avec un flux d'azote. La solution obtenue a été stérilisée à 121 °C pendant 15 minutes, puis elle a été répartie dans des flacons stériles et apyrogènes fermés avec des bouchons caoutchouc et des capsules en aluminium, lavés et passés à l'autoclave.

### Exemple 2 : Etude clinique d'efficacité chez les ruminants des compositions vétérinaires à base de cabergoline

Cette étude a pour objectif d'évaluer la baisse de la production laitière chez 8 vaches multipares de race Prim'Holstein en lactation depuis au moins 6 mois. Ces vaches sont âgées de 3 à 9 ans et ont un niveau de production variant de 20 à 31 kg par jour au démarrage de l'étude. L'étude était organisée selon un design en cross-over (2 traitements x 2 périodes).

La formulation décrite dans l'Exemple 1, contenant 500 mcg/ml de cabergoline en solution injectable a été utilisée dans cette étude.

**Traitement Test** (C646): solution injectable de cabergoline à 500 mcg/ml, 1 injection unique intra musculaire à la dose de 5 mcg/kg soit 1 ml pour 100 kg de poids vif.

**Traitement Témoin** (Placebo) : eau pour préparation injectable, 1 injection intramusculaire à un volume identique à celui du traitement Test soit 1 ml pour 100 kg.

La séquence de traitement a été randomisée (A-B ou B-A) avec une période de wash-out de 3 semaines entre les 2 traitements. La production laitière individuelle a été suivie à chaque traite sur une période allant de 7 jours avant traitement à 7 jours après traitement. Des prélèvements sanguins ont été réalisés quotidiennement afin de suivre l'évolution des concentrations plasmatiques de prolactine, sur une période allant de 2 jours avant traitement à 7 jours après traitement.

Les résultats représentés dans la Figure 1, montrent que l'injection du traitement Test à la dose de 5 mcg/kg entraînait une réduction de la production laitière :
- Dès la 1^{ère} traite suivant l'injection du traitement Test (12h), la réduction de la production de lait était en moyenne de 27%, pouvant aller jusqu'à 42% selon les individus.
- sur la période de suivi de 7 jours après traitement Test, la réduction globale de la production lait était en moyenne de 15 %, pouvant aller jusqu'à 27% selon les individus.

En généralisant les résultats à une population de vache, ces résultats permettent d'estimer que la réduction de production laitière dès la 1^{ère} traite varie entre 5 et 60% selon les individus. La réduction de la production laitière après injection du traitement Test est vraisemblablement comprise entre 25 et 35% dès la traite suivante. Cette diminution a été maintenue pendant environ 2 jours. Une reprise progressive de la production de lait a été ensuite observée, sans toutefois retrouver son niveau initial après 1 semaine.

Parallèlement les concentrations plasmatiques en prolactine ont diminué comme le montre la Figure 2. La diminution de la concentration plasmatique en prolactine a été rapide, dès 24 h suivant l'injection, et était encore présente une semaine après traitement. Cette baisse de prolactine varie de 10 à 80% selon les individus.

L'administration unique de cabergoline a mise en évidence la corrélation entre la baisse significative de la production de lait à la baisse de la prolactine plasmatique.

### Exemple 3 : Etude clinique de sécurité chez les ruminants de compositions

### vétérinaires à base de cabergoline

Cette étude vise à montrer l'absence de risque abortif et de foeto-toxicité lors d'utilisation de la cabergoline chez des ruminants gestants même dans le cas d'un surdosage de la composition vétérinaire à base de carbergoline par rapport à la posologie prescrite selon la présente invention. Des groupes de vaches à différents stades de gestation reçoivent le traitement à différentes doses :
- 3 mois de gestation (6 animaux), dose de 15 mcg/kg - 2 injections à 48 heures d'intervalle
- 7 à 8 mois de gestation : (5 animaux), dose de 15 mcg/kg - 2 injections à 48 heures d'intervalle
- 6.5 à 7.5 mois de gestation : (11 animaux), dose de 25 mcg/kg - 2 injections à 48 heures d'intervalle

La formulation décrite dans l'Exemple 1, contenant 500 mcg/ml de cabergoline en solution injectable est utilisée dans cette étude.

Des examens cliniques réguliers sont réalisés sur une période allant de 7 jours avant traitement à 15 jours après traitement :
- Surveillance quotidienne clinique générale et des avortements,
- Examen clinique du tractus génital incluant une palpation transrectale et une échographie, afin d'évaluer la souffrance et la viabilité foetale.

Des prélèvements sanguins sont réalisés 1 jour avant le traitement et 15 jours post traitement pour suivre les concentrations plasmatiques de différentes hormones et protéines indicatrices de la santé du placenta et du foetus, telles que la progestérone, le sulfate d'oestrone, et la PAG (Pregnancy Associated Glycoprotein). Les animaux sont suivis jusqu'à la mise bas afin d'évaluer l'état de santé des veaux nouveau-nés.

Les résultats montrent que deux injections de cabergoline à 48 h d'intervalles jusqu'à la dose de 25 mcg/kg ne provoquent pas d'avortement, ni d'effet néfaste sur la gestation, la viabilité et la santé des veaux nouveau-nés. Aucune variation des hormones indicatrices d'une toxicité placentaire ou foetale en relation avec les traitements aux différentes doses n'a été observée.

Par ailleurs, sur un plan général, les animaux ont bien toléré les traitements. En conclusion, une administration unique ou répétée à 48 heures de cabergoline jusqu'à 25 mcg/kg au moins est sans risque chez la vache gestante.

### Exemple 4 (exemple de référence)

### : Etude clinique d'efficacité et de sécurité de l'avortement chez les ruminants des compositions vétérinaires à base de quinagolide:

9 vaches multipares de race Prim'Holstein (environ 600 kg) gestantes d'environ 7 mois avec une niveau de production laitière au environ de 18 kg sont inclues dans l'étude. L'étude est organisée en « cross-over » : 2 traitements x 2 périodes, avec une période de « wash-out » de 3 semaines.

Les animaux reçoivent le traitement test par voie intramusculaire à partir de formulations injectables adaptées.

**Traitement Test :** solution injectable de 0.5 mg/ml de quinagolide, 1 injection unique intra musculaire à la dose de 1 à 3 mg/vache soit 1 ml pour 100 kg de poids vif.

**Traitement Témoin** (Placebo) : eau pour préparation injectable, 1 injection intramusculaire à un volume identique à celui du traitement Test soit 1 ml pour 100 kg.

Le suivi de la production laitière est organisé 7 jours avant et après le jour du traitement pour chaque période, ainsi qu'une surveillance des avortements similaire à celle décrite dans l'exemple 3 est réalisée pendant toute la durée de l'étude.

### Exemple 5 (exemple de rérérence)

### : Etude clinique d'efficacité et de sécurité de l'avortement chez les ruminants des compositions vétérinaires à base de bromocriptine :

9 vaches multipares de race Prim'Holstein (environ 600 kg) gestantes d'environ 7 mois avec un niveau de production laitière au environ de 18 kg sont inclues dans l'étude. L'étude est organisée en « cross-over »: 2 traitements x 2 périodes, avec une période de « wash-out » de 3 semaines.

Les animaux reçoivent les traitements test par voie intramusculaire à partir de formulations injectables adaptées.

**Traitement Test** (bromocriptine): solution injectable de 10 mg/ml de bromocriptine, 1 injection unique intra musculaire à la dose de 100 à 200 mcg/kg soit 1 ml pour 100 à 50 kg de poids vif.

**Traitement Témoin** (Placebo) : eau pour préparation injectable, 1 injection intramusculaire à un volume identique à celui du traitement Test soit 1 ml pour 100 kg. Le suivi de la production laitière est organisé 7 jours avant et après le jour du traitement pour chaque période, ainsi qu'une surveillance des avortements similaires à celle décrite dans l'exemple 3 est réalisée pendant toute la durée de l'étude.

## Revendications

1. Composition vétérinaire comprenant de la cabergoline pour une utilisation pour induire un tarissement de la lactation et favoriser l'involution mammaire chez des ruminants en gestation, de manière à favoriser leur bien-être ou à traiter et/ou prévenir des maladies et/ou infections de leurs mamelles.

2. Composition pour une utilisation selon la revendication 1, **caractérisée en ce que** ladite composition est administrée en une dose suffisante pour induire un tarissement ou une baisse de la lactation compris entre 5 et 60%, 20 et 50%, ou entre 25 et 35%.

3. Composition pour une utilisation selon la revendication 1 ou 2, **caractérisée en ce que** ladite composition est administrée à une dose efficace sans entraîner d'effet délétère ou abortif sur la gestation des ruminants.

4. Composition pour une utilisation selon l'une quelconque des revendications 1 à 3, **caractérisée en ce que** ladite composition est administrée par voie parentérale, cutanée ou orale.

5. Composition pour une utilisation selon l'une quelconque des revendications 1 à 4, **caractérisée en ce que** ladite composition est administrée par voie injectable, plus particulièrement en une seule fois par injection intramusculaire, ou encore par injection sous-cutanée.

6. Composition pour une utilisation selon l'une quelconque des revendications 1 à 5, **caractérisée en ce que** les ruminants sont des mammifères herbivores choisis parmi les bovins, les ovins, les caprins, les camélidés, ou les bovidés.

7. Composition pour une utilisation selon l'une quelconque des revendications 1 à 6, **caractérisée** en ladite composition est administrée en une seule dose efficace par ruminant et par traitement.

8. Composition pour une utilisation selon l'une quelconque des revendications 1 à 7, **caractérisée en ce que** la baisse de la lactation ou le tarissement de la lactation est obtenue jusqu'à 7 jours après traitement avec ladite composition ou intervient dès la première traite et la traite suivante des ruminants traités.

9. Kit à usage vétérinaire comprenant une composition vétérinaire comprenant de la cabergoline, pour une utilisation pour favoriser le bien-être, favoriser l'involution mammaire, ou pour traiter et/ou prévenir les occurrences liées aux maladies des pis, des ruminants en gestation.

10. Kit pour une utilisation selon la revendication 9, pour le traitement et/ou la prévention des mammites des ruminants en gestation.

## Patentansprüche

1. Tiermedizinische Zusammensetzung umfassend Cabergolin zur Verwendung, um ein Versiegen der Laktation zu induzieren und die Involution der Milchdrüse bei trächtigen Wiederkäuern zu fördern, um so deren Wohlbefinden zu fördern oder Krankheiten und/oder Infektionen ihrer Euter zu behandeln oder zu verhindern.

2. Zusammensetzung zur Verwendung gemäß Anspruch 1, **dadurch gekennzeichnet, dass** besagte Zusammensetzung in einer hinreichenden Dosis verabreicht wird, um ein Versiegen oder einen Rückgang der Laktation zwischen 5 und 60%, 20 und 50% oder zwischen 25 und 35% zu induzieren.

3. Zusammensetzung zur Verwendung gemäß Anspruch 1 oder 2, **dadurch gekennzeichnet, dass** besagte Zusammensetzung mit einer wirksamen Dosis verabreicht wird, ohne eine schädliche oder abortive Wirkung auf die Trächtigkeit von Wiederkäuern zur Folge zu haben.

4. Zusammensetzung zur Verwendung gemäß einem der Ansprüche 1 bis 3, **dadurch gekennzeichnet, dass** besagte Zusammensetzung parenteral, perkutan oder oral verabreicht wird.

5. Zusammensetzung zur Verwendung gemäß einem der Ansprüche 1 bis 4, **dadurch gekennzeichnet, dass** besagte Zusammensetzung mittels Injektion, insbesondere in einer einzigen intramuskulären Injektion oder auch subkutanen Injektion, verabreicht wird.

6. Zusammensetzung zur Verwendung gemäß einem der Ansprüche 1 bis 5, **dadurch gekennzeichnet, dass** die Wiederkäuer herbivore Säugetiere sind, ausgewählt aus Rindern, Schafen, Ziegen, Kamelen oder Horntieren.

7. Zusammensetzung zur Verwendung gemäß einem der Ansprüche 1 bis 6, **dadurch gekennzeichnet, dass** besagte Zusammensetzung in einer einzigen wirksamen Dosis je Wiederkäuer und je Behandlung verabreicht wird.

8. Zusammensetzung zur Verwendung gemäß einem der Ansprüche 1 bis 7, **dadurch gekennzeichnet, dass** der Rückgang der Laktation oder das Versiegen der Laktation in bis zu 7 Tagen nach Behandlung mit besagter Zusammensetzung erhalten wird oder bereits bei dem ersten Melken und dem darauffolgenden Melken der behandelten Wiederkäuer eintritt.

9. Kit zur tiermedizinischen Nutzung umfassend eine tiermedizinische Zusammensetzung umfassend Cabergolin zur Verwendung zur Förderung des Wohlbefindens, Förderung der Involution der Milchdrüse oder für die Behandlung und/oder Prävention von Phänomenen, die mit Euterkrankheiten von trächtigen Wiederkäuern verbunden sind.

10. Kit zur Verwendung gemäß Anspruch 9 für die Behandlung und/oder Prävention von Euterentzündungen von trächtigen Wiederkäuern.

## Claims

1. A veterinary composition comprising cabergoline for a use to induce lactation depletion and promoting mammary involution in gestating ruminants.

2. The composition for a use according to the claim 1, wherein the composition is administered in an effective amount so as to induce depletion or reduction of lactation comprised between 5 and 60%, 20 and 50%, or between 25 and 35%.

3. The composition for use according to any of claims 1 to 2, wherein the composition is administered in an effective amount without causing any deleterious or abortive effects on the gestating ruminants.

4. The composition for use according to any of claims 1 to 3, wherein the composition is administered via parenteral, cutaneous or oral route.

5. The composition for use according to any of claims 1 to 4, wherein the composition is administered via injection, more particularly as single administration via intramuscular injection, or via subcutaneous injection.

6. The composition for use according to any of claims 1 to 5, wherein the ruminants are herbivorous mammals selected from bovines, ovines, goats, camelids or bovids.

7. The composition for use according to any of claims 1 to 6, wherein the composition is administered in one single effective dose per ruminant and per treatment.

8. The composition for use according to any of claims 1 to 7, wherein the reduction of lactation or lactation depletion is obtained up to 7 days after the treatment with the said composition or occurs at the first milking and next milking of the treated ruminants.

9. A kit for a veterinary use comprising a veterinary composition comprising cabergoline, for a use for improving the welfare, for promoting mammary involution or for treating and/or preventing occurrences linked to intra-mammary diseases, of gestating ruminants.

10. The kit for a use according to claim 9, for treating and/or preventing mastitis of gestating ruminants.
